# EUROPEAN PATENT SPECIFICATION

(11) **EP 0 661 059 B1**
(45) Date of publication and mention of the grant of the patent: **28.11.2001**
(21) Application number: 94119949.9
(22) Date of filing: 16.12.1994
(51) Int. Cl.: A61K 39/295

(54) **An intranasal bovine trivalent vaccine containing modified live IBRV, PI3V and BRSV**
Ein intranasaler trivalenter Rinderimpfstoff der modifiziertes lebendes IBRV, PI3V und BRSV enhält
Un vaccin trivalent bovin intranasal contenant IBRV, PI3V et BRSV vivants et modifiés

(30) Priority: 29.12.1993 US 175093
(43) Date of publication of application: 05.07.1995
(73) Proprietor: Bayer Corporation, Pittsburgh, PA 15205-9741 (US)
(72) Inventor: Ciszewski, Daniel K., Carlisle, PA 50047 (US); McGinley, Michael J., Lenexa, Kansas 66216 (US); Phillips, Connie S., Des Moines, IA 50310 (US); Schnurr, Michael J., Ankeny, IA 50021 (US)
(74) Representative: Linkenheil, Dieter

(56) References cited:
- WO-A-95/30437
- JOURNAL OF THE AMERICAN VETERINARY MEDICAL ASSOCIATION, vol. 194, no. 9, May 1989, pages 1273-1280, XP000568596 L.T. TALENS ET AL.: "EFFICACY OF VIRAL COMPONENTS OF A NONABORTIGENIC COMBINATION VACCINE FOR PREVENTION OF RESPIRATORY AND REPRODUCTIVE SYSTEM DISEASES IN CATTLE"
- VETERINARY RECORD, vol. 119, 1986, pages 450-453, XP000567788 J.R. THOMSON ET AL.: "A BOVINE RESPIRATORY VIRUS VACCINATION TRIAL"
- DEVELOP. BIOL. STAND., vol. 28, 1975, pages 482-488, XP000568335 N. ZYGRAICH ET AL.: "LOCAL AND SYSTEMIC RESPONSE AFTER SIMULTANEOUS INTRANASAL INOCULATION OF TEMPERATURE-SENSITIVE MUTANTS OF PARAINFLUENZA 3, IBR AND BOVINE ADENOVIRUS"

## Description

### BACKGROUND OF THE INVENTION

### Field of the Invention:

The present invention relates to the use of certain viruses for the preparation of a vaccine for protecting cattle against viral mediated respiratory diseases.

### Brief Description of the Prior Art:

It is believed that the viruses comprising infectious bovine rhinotracheitis virus (IBRV), parainfluenza subtype 3 (PI₃V) and bovine respiratory syncitial virus (BRSV) are significant contributors to the respiratory disease complex in cattle. Disease signs of IBRV may include high fever, hyperpnea, dyspnea and severe inflammation of the nasal mucosa with formation of mucoid plaques. Disease signs caused by PI₃V may be weakness, depression, watery to mucopurulent nasal discharge, fever, coughing and weight loss. Disease signs of BRSV may include fever, cough, nasal discharge, ocular discharge, anorexia, hyperpnea, pulmonary edema and emphysema, and denuding of the ciliated epithelium, leading to secondary bacterial pneumonia and associated sequela. The signs vary in severity, and can progress rapidly to a crisis phase.

To protect bovines against the viral components of the respiratory disease complex, it has been known in the art to intramuscularly or subcutaneously administer to bovines a trivalent vaccine(s) containing a modified live infectious bovine rhinotracheitis virus (IBRV), parainfluenza subtype 3 (PI₃V) and bovine respiratory syncitial virus (BRSV) in a single vaccine.

Talens et al. (Journal of the American Veterinary Medical Association, Vol. 194, No. 9, May 1989, pages 1273-1280) describe an IM-administered multivalent vaccine comprising among others modified live IBR, Pl₃ and BRS viruses.

While it is recognized that mucosal administration, orally or intranasally, would be a more effective mode of administration, the art has heretofore not developed a safe and effective trivalent vaccine and a method of administering such vaccine containing these viruses, intranasally.

Safe intranasally administered IBRV vaccines have been described. Zygraich and Lobmann (U.S. Patent No. 3,907,986) have described a mutant strain of IBRV prepared by: a) induction and isolation of temperature sensitive mutant strains and b) serial passage of the obtained temperature-sensitive strains in heterologous cells. This process was used to produce an IBRV vaccine which was safe when administered intranasally to cattle. However, this group did not combine their IBRV mutant with other viruses of interest here to show that it was still safe and that other viruses do not adversely affect the mutant IBRV. Incidentally, Zygraich et al (Dev. Biol. Stand. 28: 482-488) have demonstrated that simultaneous application of modified live IBRV, PI₃V and Adenovirus Type 3 did not produce disease when administered intranasally to susceptible cattle. These scientists did not evaluate BRSV as an added component to this vaccine. They mentioned that they failed to demonstrate IBRV antibody after a single intranasal application. It took two intranasal applications to stimulate a high antibody level.

As is presently known, a trivalent combination of IBRV, PI₃V and BRSV strains which were modified live and appear to be safe and effective when administered by the intramuscular route, produced disease when administered intranasally. It is speculated that the intranasal route of administration is equivalent to the normal infection route and, therefore, virus strains which are not "sufficiently mutated" will produce disease. By the term "sufficiently mutated" is meant modifying the virus adequately to allow intranasal administration of the virus in a live form to cattle without producing adverse effects will produce disease.

Heretofore no trivalent vaccine prepared from modified live IBRV, PI₃V and BRSV has been known to be safe and effective in bovines when administered by the intranasal route. Without being bound to one particular theory it is believed that this vaccine combination has not been feasible because it has either demonstrated untoward reactions post vaccination (such as production of disease) or has demonstrated an interference interaction between one or more of the viruses such that the antigenicity is significantly reduced.

From the foregoing, it would be realized that there is a need for a trivalent vaccine containing IBRV, PI₃V and BRSV and a method of intranasally administering said vaccine to bovines.

### SUMMARY OF THE INVENTION

In accordance with the following, the present invention encompasses the use of an intranasally non-virulent modified live infectious bovine rhinotracheitis virus (IBRV), a modified live bovine parainfluenza subtye 3 (Pl₃V) and a non-virulent bovine respiratory syncitial virus (BRSV) for preparing a trivalent vaccine for protecting cattle against viral mediated respiratory diseases, wherein the vaccine can be administered intranasally in amounts that would protect cattle without causing vaccine-induced adverse effects including signs of disease.

Surprisingly, it has been found by the present invention that there is no interference between the viruses and that bovine hosts responded by developing protective titers after intranasal administration of the vaccines to them.

### DETAILED DESCRIPTION OF THE INVENTION

The trivalent vaccine useful herein contains modified live, "intranasally non-virulent", infectious bovine rhinotracheitis virus (IBRV), modified live parainfluenza subtype 3 (PI₃V) and non-virulent bovine respiratory syncitial virus (BRSV). By the term "modified live" is meant the virus has been reduced in virulence by any of several methods known in the art such as:
1) repeated passage in cell culture; 2) forced adaptation to growth at normally-restrictive temperatures; 3) treatment with chemical mutagens to force high numbers of mutations and selection for the desired characteristics; and 4) deletion or insertion of genes using rDNA technology. By the term "intranasally non-virulent" is meant the modified live virus exhibits reduced or no clinical signs of infection when administered intranasally. As would be realized, the components of the vaccine are compatible and do not interfere with each other by blocking immunization potential and together are believed to constitute a safe and effective multi-antigenic vaccine that protects bovine hosts against viral mediated respiratory disease. In the preparation of the vaccine, the antigens of the respective viruses are combined in a proper ratio and stabilized.

Illustratively, the vaccine was prepared according to the following methods.. The IBRV component (original isolate) of the vaccine was obtained from a field case of infectious bovine rhinotracheitis. The strain was modified to reduce virulence by multiple passage in primary bovine kidney cells followed by 18 passages in primary rabbit kidney cells. The thus-modified live IBRV strain was used to prepare a Master Seed by passaging it 18 times in primary rabbit kidney cells in accordance with art-known techniques for the preparation of a modified live virus which serves as the Master Seed Virus. A Working Seed was prepared from the Master Seed by passaging the Master Seed in primary rabbit kidney cells four more times. The 1st to 4th passage are used as Working Seed for actual vaccine production.

Virus from the Working Seed was used to prepare the vaccine by combining approximately 10^{6.0} TCID₅₀ (Tissue Culture Infective Dose₅₀) equivalents of virus/ml with an industry standard stabilizer (TCID₅₀ means the dilution of virus that produces a cytopathic effect (CPE) in 50% of the wells of a tissue culture plate).

Stabilized viral fluids were then cryopreserved by standard freeze-drying (lyophilization) methods.

The PI₃V useful herein is known in the art. The original strain was obtained and isolated from a respiratory outbreak in 1962. This original isolate was used to prepare a Master Seed by passaging it fifteen times in primary bovine kidney cells and nine times in porcine kidney cells in accordance with art-known techniques for the preparation of a modified live virus which serves as the Master Seed Virus. A Working Seed was prepared from the Master Seed by passaging the master seed in bovine kidney cells an additional 4 times. These four passages all serve as Working Seed. Virus from the working seed was used to prepare vaccine by combining approximately 10^{6.5} TCED₅₀ equivalents/ml of virus with an industry standard stabilizer. Stabilized viral fluids were then cryopreserved by standard freeze-drying methods.

As to the BRSV useful herein, the original seed strain was also obtained and isolated from a field case of BRSV. The original seed strain was used to prepare a Master Seed by passaging it seven times in bovine kidney cells, eight times in bovine turbinate cells, and forty-six additional times in bovine kidney cell cultures in accordance with art-known techniques for the preparation of a modified live virus. A Working

Seed was prepared from the Master Seed by passaging the Master Seed four more times in bovine kidney cells. These four passages all serve as Working Seed.

Virus from the Working Seed was used to prepare the vaccine by combining approximately 10^{5.1} TCID₅₀ equivalents/ml of virus with an industry standard stabilizer. Stabilized viral fluids were then cryo-preserved by standard freeze-drying methods.

As would be realized from the foregoing description, the useful viruses are known in the art and methods of growing the viruses such as propagation in a cell culture are also known in the art. Illustratively, the method of propagating the respective viruses in a cell culture comprises inoculating cells therewith, incubating the cells until a cytopathic effect is realized, harvesting the propagated virus, followed by freeze-thawing the harvested virus and collecting the propagated virus.

In accordance with the invention, the viruses employed herein are in a modified live form. Methods of preparing modified live viruses are known in the art. It is a distinct feature of the invention that the modified live viruses useful herein are characterized in that they are intranasally non-virulent. Methods of making a modified live virus intranasally, non-virulent would be within the purview of the skilled artisan. For example, a modified live virus can be made intranasally non-virulent by multiple passage in host and/or non-host cell culture, by forced adaptation to growth at normally-restrictive temperatures, by chemical mutagenesis followed by selection of desired characteristics, and by deletion or addition of genes by rDNA techniques.

The viruses can be combined into a trivalent vaccine by stabilizing the respective viruses separately before they are combined or after they have been combined. The amounts of each virus which can be used are irrelevant as long as the minimal titer levels are obtained in the final product. The viruses can be combined in any order. The stabilizer is added to the bulk vaccine before freeze-drying.

Stabilizers are known in the art to contain sugars such as sucrose, lactose and trehalose, proteins such as gelatin, serum, bovine serum albumin and NZamine, and reducing agents such as thiosulfate and glutathione.

The vaccine can be administered intranasally in a dose of from 1.0 ml to 5.0 ml, preferably from 1.0 ml to 2.0 ml. Typically the vaccine would contain between 10^{5.5} and 10^{6.5} TCID₅₀/dose of IBRV, 10^{3.5} and 10^{5.0} TCID₅₀/dose PI₃V and 10^{3.2} and 10^{4.8} TCED₅₀/dose BRSV. The lower titer levels listed indicate the minimal amount of modified live virus which has been shown to protect cattle in previously-conducted vaccination/challenge studies.

The invention is further illustrated but is not intended to be limited by the following examples in which all parts and percentages are by weight unless otherwise specified.

### EXAMPLES

A modified live intranasal vaccine made up of IBRV and PI₃V labeled Nasalgen IP® was combined with a modified live BRSV vaccine labeled BRSV Vac® to produce an experimental, intranasal (IN), trivalent (IBRV-PI₃V-BRSV), modified live virus vaccine. Reconstitution of such vaccines involved adding the 20 ml of sterile diluent (water for injection) packaged with the BRSV Vac® to the lyophilized product and shaking the contents until dissolution occurred. The reconstituted contents of the BRSV Vac® were then added to the 10 dose (20 ml) lyophilized Nasalgen IP® to reconstitute it. The final product was the trivalent vaccine (IBRV-PI₃V-BRSV).

This experimental trivalent vaccine was titrated after combination (prior to administration to cattle) in order to determine whether any interference could be detected between the various viral components and to confirm that protective levels of the three viruses were present prior to vaccination. Virus titers measured after reconstitution of the components are compared with the original allowable titers in Table 1.

**Table 1**

| **Virus titers before and after combination into a trivalent vaccine** | | |
|---|---|---|
| **Virus** | **Original Titer (TCID**^{**50**}**/ml)** | **Titer after reconstitution (TCID**_{**50**}**/ml)** |
| IBRV | 10^{5.5} TO 10^{6.5} | 10^{5.7} |
| PI₃V | 10^{3.5} TO 10^{5.0} | 10^{3.8} |
| BRSV | 10^{3.2} TO 10^{4.8} | 10^{3.9} |

As mentioned earlier, an IBR titer of at least 10^{5.5} TCID₅₀/ml has been shown to protect cattle from challenge with virulent virus. The reconstituted trivalent vaccine contained 10^{5.7} TCID₅₀/ml which falls above the minimum protective level required and indicates that the other two virus components do not interfere with this fraction. Additionally, the trivalent vaccine contained 10^{3.8} TCID₅₀/ml of PI₃V which is also above the minimum protective level for this virus. Therefore, the IBRV and BRSV do not interfere with the PI₃V. Finally, the BRSV titer in the reconstituted trivalent vaccine was 10^{3.9} TCID₅₀/ml which is above the minimum protective dose for this virus and indicates that the IBRV and PI₃V do not interfere with BRSV. It is apparent that this trivalent combination overcomes the problem of incompatibility or interference between these three viruses.

In order to confirm the lack of interference and prove the effectiveness of the trivalent vaccine in the host, calves were vaccinated with the trivalent vaccine and compared with calves vaccinated with the individual vaccines (Nasalgen IP® and BRSV VacR®) as well as with non-vaccinated control calves. These comparisons involved observing the animals for disease or adverse reactions post vaccination and evaluating the serum neutralizing antibody titers developed by the cattle for both IBRV and BRSV, the two most critical fractions in this trivalent combination.

Twenty-five calves were split into five groups of five animals each and treated as described in Table 2. Trivalent vaccine was administered to 5 calves in a 1.0 ml per nostril dose which is considered the normal field dose (1X). Five cattle received five times (5X) the normal field dose in order to confirm the safety of the modified live trivalent intranasal vaccine. As controls, the Nasalgen IP® and BRSV VacR® were administered to 5 calves each and one group of 5 calves received no vaccine.

**Table 2**

| **Vaccination regimen for calves** | | | |
|---|---|---|---|
| **Group** | **No. of Calves** | **Vaccine** | **Dosage ml/Nostril** |
| 1 | 5 | Trivalent (IBRV-Pl₃V-BRSV) | 1X - 1.0 |
| 2 | 5 | Trivalent (IBRV-Pl₃V-BRSV) | 5X - 5.0 |
| 3 | 5 | Nasalgen IP® (IBRV-Pl₃V) | 1X - 1.0 |
| 4 | 5 | BRSV VacR® (BRSV) | 1X - 1.0 |
| 5 | 5 | Nonvaccinated Controls | none |

| | | | |
|---|---|---|---|
| 1X = Field Dose | | | |
| 5X = Five Times The Field Dose | | | |

All calves were observed post vaccination for signs of disease (nasal erosions, ocular discharge, nasal discharge, anorexia, coughing and elevation of rectal temperature).

Points were assigned to clinical signs as indicated below:
Normal = Z = 0 points/day
Nasal Discharge = N = 1 point/ day
Ocular Discharge = 0 = 1 point/day
Coughing = C = 2 points/day

Results of clinical observations are shown in Table 3.

Table 3 not only shows daily clinical signs but also summarizes the 14-day results numerically under the column marked total. It is noted that the nonvaccinated control calves responded with a total average clinical sign score of 1.2. This can be considered a baseline. Groups 1, 3, and 4 calves had clinical sign score totals averaging less than that of the nonvaccinated controls. Only group 2 calves had a clinical sign average greater than that of the nonvaccinated control group. Even this value of 4.6 is not a clear indication of disease as calves which receive virulent virus routinely show a clinical sign average of 25 or higher. It is, therefore, concluded that all of the vaccines tested herein, including the trivalent vaccine containing five times the normal level of the three viruses, were safe in calves when administered intranasally.

The final proof of compatibility of IBRV, PI₃V and BRSV in an intranasal trivalent vaccine is demonstrated by measuring the antibody responses in the calves receiving the various vaccines described previously (Table 2). Antibody responses were quantitated using serum neutralization assays known in the art. Such assays involve mixing diluted serum from animals in the study with a standard amount of virulent virus and then placing the mixture on a susceptible tissue culture. If the virus has not been neutralized by antibodies in the serum the tissue culture cells will show a cytopathic effect (CPE). If antibody is present in the serum dilution it will block the CPE. The last dilution of serum to completely neutralize the virus is considered the endpoint and is denoted the antibody titer. In this study, one must compare the antibody titers produced by calves vaccinated the 1X trivalent vaccine with calves vaccinated with Nasalgen IP® and BRSV Vac®. Such a comparison will demonstrate whether there was a loss of antigenicity when the trivalent vaccine was formulated.

Table 4 lists the antibody titers against IBRV and BRSV on the day of vaccination (Day 0) and at 14 days post vaccination. Since PI₃V seronegative calves are almost impossible to find and this viral component is known to be stable, antibody titers against it were not evaluated. Although the individual calf titers are listed, the geometric mean titer (GMAT) is the most meaningful and should be compared for the group response. It is noted in Table 4, that the Nasalgen IP® and BRSV Vac® vaccines (Groups 3 and 4) produced 8-fold and 3-fold increases in IBRV titers respectively; whereas, the trivalent 1X (Group I) vaccine produced a 28.5-fold increase in IBRV titer and the trivalent 5X (Group II) vaccine produced a 22.6-fold increase in IBRV titer. These are truly vaccine responses as the nonvaccinated controls (Group 5) did not show an increase in titer. Obviously, the IBRV was not adversely affected by the other two virus components in this trivalent combination vaccine. Similar titer trends are observed with the BRSV fraction. The BRSV virus is known to generally produce low antibody titers. The antibody responses produced by calves receiving the trivalent 1X and trivalent 5X vaccines (2.3-fold and 2.4-fold respectively) were comparable to those titers produced by calves receiving Nasalgen IP® and BRSV Vac® R (1.6-fold and 3.3-fold respectively). One can conclude that the BRSV was not adversely affected by IBRV or PI₃V. Again, the nonvaccinated control calves showed no increase in antibody titer indicating that there was no external exposure to BRSV.

Surprisingly, the intranasal vaccination of calves with this newly-discovered trivalent vaccine containing IBRV, PI₃V and BRSV produced a significant antibody response which would normally be considered protective. Additionally, this combination trivalent vaccine, even when administered intranasally as a 5X dose, did not produce adverse signs in calves post vaccination indicating that the combination is safe.

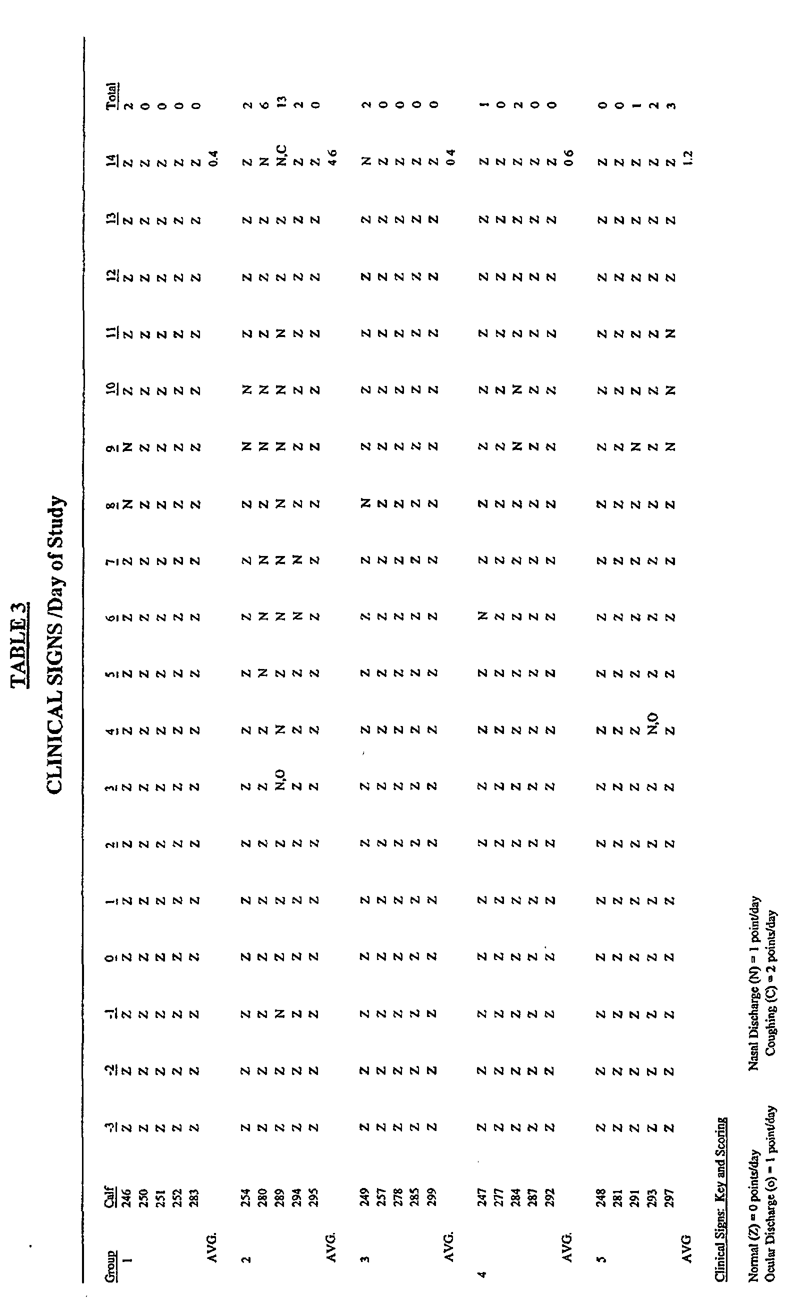

**Table 4**

| **IBR AND BRSV SERUM ANTIBODY TITERS** | | | | | |
|---|---|---|---|---|---|
| | | **IBR** | | **BRSV** | |
| **Group** | **Calf** | **Day 0** | **Day 14** | **Day 0** | **Day 14** |
| 1 | 246 | < 2 | 45 | 6 | 8 |
| | 250 | < 2 | 32 | 16 | 8 |
| | 251 | 2 | 45 | <2 | <8 |
| | 252 | <2 | 45 | 16 | 45 |
| | 283 | <2 | 16 | 3 | 16 |
| | GMAT | 1.2 | 34.2 | 5.4 | 12.6 |
| | | | | | |
| 2 | 254 | <2 | 16 | 6 | 11 |
| | 280 | <2 | 16 | 8 | 32 |
| | 289 | <2 | 90 | 4 | 8 |
| | 294 | <2 | 16 | 3 | 11 |
| | 295 | <2 | 16 | 8 | 11 |
| | GMAT | 1.0 | 22.6 | 5.4 | 12.7 |
| | | | | | |
| 3 | 249 | <2 | 11 | 11 | 32 |
| | 257 | 3 | 4 | 4 | 8 |
| | 278 | 3 | 45 | 11 | 16 |
| | 285 | <2 | 16 | 8 | 11 |
| | 299 | <2 | 11 | 8 | 8 |
| | GMAT | 1.6 | 12.8 | 7.9 | 12.9 |
| | | | | | |
| 4 | 247 | <2 | <4 | 4 | 11 |
| | 277 | <2 | <4 | <2 | 8 |
| | 284 | <2 | <4 | 3 | 8 |
| | 287 | <2 | <4 | <2 | <8 |
| | 292 | <2 | <4 | 16 | 16 |
| | GMAT | 1.0 | 3.0 | 2.9 | 9.5 |
| | | | | | |
| 5 | 248 | <2 | <2 | <2 | 2 |
| | 281 | <2 | 2 | 11 | 3 |
| | 291 | <2 | <2 | 6 | 4 |
| | 293 | 3 | <2 | 8 | 2 |
| | 297 | <2 | <2 | 3 | <2 |
| | GMAT | 1.3 | 1.2 | 4.4 | 2.2 |

## Claims

1. Use of an intranasally non-virulent modified live infectious bovine rhinotracheitis virus (IBRV), a modified live bovine parainfluenza subtype 3 (Pl₃V) and a non-virulent bovine respiratory syncitial virus (BRSV) for preparing a trivalent vaccine for protecting cattle against viral mediated respiratory diseases, wherein the vaccine can be administered intranasally in amounts that would protect cattle without causing vaccine-induced adverse effects including signs of disease.

2. The use of claim 1 wherein the vaccine is in a dosage of 1 to 5 ml.

3. The use of claim 1 wherein the IBRV, PI₃V and BRSV are present to titers of at least 10^{5,5} TCID₅₀/dose IBRV; at least 10^{3.5} TCID₅₀/dose PI₃V and at least 10^{3.2} TCID₅₀/dose BRSV.

## Patentansprüche

1. Verwendung eines intranasalen nicht-virulenten modifizierten infektiösen Lebend-Rinder-Rhinotracheitis-Virus (IBRV), eines modifizierten Lebend-Rinder-Parainfluenza-Subtyp 3 (PI₃V) und eines nicht-virulenten Rinder-Respiratorsynzitial-Virus (BRSV) zur Herstellung eines trivalenten Impfstoffes zum Schutz von Vieh vor viral mediierten Atmungskrankheiten, wobei der Impfstoff intranasal in Mengen verabreicht werden kann, die das Vieh schützen, ohne Impfstoff-induzierte Gegeneffekte, einschließlich von Krankheitsanzeichen, zu verursachen.

2. Verwendung gemäß Anspruch 1, wobei der Impfstoff in einer Dosierung von 1 bis 5 mL vorhanden ist.

3. Verwendung gemäß Anspruch 1, wobei das IBRV, PI₃V und das BRSV mit Titern von mindestens 10^{5,5} TCID₅₀/Dosis IBRV, mindestens 10^{3,5} TCID₅₀/Dosis PI₃V und von mindestens 10^{3,2} TCID₅₀/Dosis BRSV vorhanden sind.

## Revendications

1. Utilisation d'un virus de la rhino-trachéite infectieuse des bovidés (IBRV) vivant modifié, non virulent de manière intranasale, d'un virus para-influenza de sous-type 3 (PI₃V) de bovidés vivant modifié et d'un virus syncitial respiratoire bovin (BRSV) non virulent, pour préparer une vaccin trivalent pour protéger le bétail contre les maladies respiratoires virales, où le vaccin peut être administré de manière intranasale en des quantités qui protègent le bétail sans provoquer d'effets nuisibles induits par le vaccin, y compris des signes de maladie.

2. Utilisation suivant la revendication 1, dans laquelle le vaccin est administré en une dose allant de 1 à 5 ml.

3. Utilisation suivant la revendication 1, dans laquelle l'IBRV, le PI₃V et le BRSV sont présents à des titres d'au moins 10^{5,5} TCID₅₀/dose d'IBRV ; d'au moins 10^{3,5} TCID₅₀/dose de PI₃V et d'au moins 10^{3,2} TCID₅₀/dose de BRSV.
